# EUROPÄISCHE PATENTSCHRIFT

(11) **EP 1 482 993 B1**
(45) Veröffentlichungstag und Bekanntmachung des Hinweises auf die Patenterteilung: **31.05.2006**
(21) Anmeldenummer: 03743819.9
(22) Anmeldetag: 27.02.2003
(51) Int. Cl.: A61L 15/07

(54) **VERFAHREN ZUR HERSTELLUNG EINER SCHLICK-SUSPENSION FÜR VERBANDSGEWEBE**
METHOD FOR THE PRODUCTION OF A MUD SUSPENSION FOR BANDAGE TISSUE
PROCEDE DE PRODUCTION D'UNE SUSPENSION DE PATE ET D'UN BANDAGE POURVUE DE CETTE SUSPENSION

(30) Priorität: 08.03.2002 DE 10210398
(43) Veröffentlichungstag der Anmeldung: 08.12.2004
(73) Patentinhaber: Norddeutsches Seebäderlabor, 27478 Cuxhafen (DE)
(72) Erfinder: KAESSMANN, Hans-Jürgen, 23570 Travemünde (DE)
(74) Vertreter: Hauck, Graalfs, Wehnert, Döring, Siemons
(86) Internationale Anmeldenummer: PCT/EP2003/001980
(87) Internationale Veröffentlichungsnummer: WO 2003/075969

(56) Entgegenhaltungen:
- DE-A- 3 221 502
- DE-A- 3 939 926
- DE-U- 20 206 044

## Beschreibung

Die Erfindung betrifft ein Verfahren zur Herstellung einer Schlick-Suspension für ein flächiges Verbandsgewebe sowie ein Verbandsgewebe mit einer Schlick-Suspension.

Fango-Kompressen für die Wärmetherapie sind seit langem bekannt. Hierbei wird feingemahlener, schwefelhaltiger Fango in Wattevliese eingestreut und mit Mull umhüllt. Die Fango-Kompressen werden in 50° bis 60° heißem Wasser erwärmt, ausgedrückt und je nach ärztlicher Anweisung für 20 bis 60 Minuten aufgelegt, wobei diese mit einem Tuch oder einer Binde festgehalten werden.

Aus der französischen Offenlegungsschrift Nr. 80 02 506 sind Breiumschläge bekannt, die ein Erzeugnis auf Basis von Meeresschlick enthalten. Der Meeresschlick wird in einem Ofen entwässert und nachfolgend pulverisiert. Vor der Anwendung wird ähnlich wie bei der Fango-Packung Wasser dem Breiumschlag zugesetzt.

Aus DE 39 39 926 ist ein flächiges Verbandsgewebe bekannt, das mit einer Suspension beschichtet ist. Als Suspension wird eine Peloid-Suspension aus Meeresschlick, Naturmoor oder Fango eingesetzt. Das so gewonnene Verbandsgewebe besitzt elastische Eigenschaften und dient als Kompressionsverband. Aus der feuchten Peloid-Suspension treten Wirkstoffe in den zu behandelnden Bereich des Körpers über und werden dort resorbiert.

Bei der Herstellung der Peloid-Suspension wurde bisher der Schlick naturbelassen verarbeitet. Nachfolgend kam es bei dem so bearbeiteten Kompressionsverband zu einem starken Schwefelwasserstoffgeruch. Dieser trat insbesondere dann auf, wenn eine Verpackung mit dem Peloid auf dem Verbandsgewebe geöffnet wurde. Auch der im feuchten Zustand aufgelegte Verband entwickelt im Verlauf seiner Trocknung einen Schwefelwasserstoffgeruch und braune bis schwarze störende Flecken.

Der Erfindung liegt die Aufgabe zugrunde eine Schlick-Suspension für ein Verbandsgewebe bereitzustellen, bei der keine Geruchsbildung auftritt und die im getrockneten Zustand eine gleichmäßige Farbe besitzt.

Erfindungsgemäß wird die Aufgabe durch ein Verfahren mit den Merkmalen aus Anspruch 1 gelöst. Vorteilhafte Ausgestaltungen des erfindungsgemäßen Verfahren bilden den Gegenstand der zugehörigen Unteransprüche.

Bei dem erfindungsgemäßen Verfahren wird Schlick auf einer flachen Unterlage verteilt und an der Luft schonend getrocknet. Die Trocknung erfolgt vorzugsweise bei Umgebungstemperatur, um eine besonders schonende Trocknung der Bestandteile zu erzielen. Der getrocknete Schlick wird nachfolgend in Stücke gebrochen, die in einer Mühle zu Schlickpulver gemahlen werden. Das Schlickpulver wird unter Zugabe von Wasser, insbesondere gereinigtem Seewasser, in einem vorbestimmten Verhältnis zu einer Suspension verrührt und die Suspension wird auf das flächige Verbandsgewebe aufgebracht. Der Vorteil dieses Verfahrens besteht darin, daß durch das Trocknen des Schlicks und die nachfolgende Herstellung einer Suspension aus Schlickpulver, es bei der Trocknung des Schlickverbandes nicht zu einer Geruchsbelästigung durch den Schwefelwasserstoffgeruch kommt. Vielmehr wurde festgestellt, daß die so gewonnene Schlick-Suspension gleichmäßig trocknet, ohne daß eine Geruchs- oder Fleckenbildung auftritt.

Darüber hinaus besitzt das erfindungsgemäße Verfahren den Vorteil, daß eine Klumpenbildung in der Schlick-Suspension vermieden wird. Bei dem bekannten Verfahren wird der naturbelassene Schlick eingesetzt, der oftmals kleine Klümpchen oder Feststoffteile besitzt. Selbst bei einem Rühren mit hoher Geschwindigkeit, lösen sich diese Bestandteile des Schlicks nicht vollständig auf, so daß es später auch bei dem Verband zu störenden Unebenheiten kommen kann, die sogar zu Druckstellen führen können. Weiterhin besitzt das erfindungsgemäße Verfahren den Vorteil, daß nach dem Trocknungsschritt der Feuchtigkeitsgehalt und der Wassergehalt sehr genau und wiederholbar bestimmt werden können.

In einer vorteilhaften Ausgestaltung ist das flächige Verbandsgewebe als ein Kompressionsverband, vorzugsweise in Form einer Binde ausgebildet. Der Kompressionsverband bietet den Vorteil, daß im feuchten Zustand aufgebracht er als ein Wirkstoff-Verband wirkt. Insbesondere besitzt ein solcher Verband mit einer Schlick-Suspension sofort nach dem Anlegen eine abschwellende Wirkung. Es wurde ebenfalls festgestellt, daß entzündliche Begleiterkrankungen seltener auftreten. Im unterkühlten Zustand wirkt der Schlick zusätzlich schmerzlindernd und hyperämisierend.

Nach ca. 2 bis 3 Stunden ist die erfindungsgemäße Schlick-Suspension so weit eingetrocknet, daß diese hart wie Gips wird. Aus dem ursprünglich aufgebrachten Wirkstoff-Verband wird also ein Stützverband. Der Stützverband besitzt gegenüber bekannten Gips- oder Zinkleimverbänden den Vorteil, daß allergische Reaktionen, wie beispielsweise Juckreiz, bisher nicht beobachtet wurden. Gegenüber den bekannten Stützverbänden kann also mit der nach dem erfindungsgemäßen Verfahren hergestellten Schlick-Suspension ein hypoallergener Stützverband bereitgestellt werden.

Bei der Herstellung des Schlickpulvers wird der Schlick in einer Dicke von ungefähr 0,5 bis 4 cm auf der Unterlage verteilt, vorzugsweise in einer Dicke von ungefähr 2 cm. Die Trocknungszeit an der Luft beträgt hierbei ungefähr ein halben bis 4 Tage. Bei einer Schichtdicke von 2 cm genügen vorzugsweise 2 bis 3 Tage zur Trocknung des Schlicks.

Je nach Art der zugesetzten Ingredienzen können diese vor oder nach dem Trocknungsprozeß dem Schlick bzw. dem Schlickpulver zugesetzt werden. Bei der Herstellung der Suspension beträgt der Wasseranteil ungefähr 30 bis 50 Gew.-%. Grundsätzlich wird als Wasser gereinigtes Meereswasser eingesetzt.

Die erfindungsgemäße Aufgabe wird ebenfalls durch ein Verbandsgewebe mit einer nach dem erfindungsgemäßen Herstellungsverfahren erzeugten Schlick-Suspension gelöst, wobei das Verbandsgewebe vorzugsweise die Form einer Binde, insbesondere einer Binde für einen Kompressionsverband aufweist.

Nachfolgend wird ein Beispiel für eine Schlick-Suspension angegeben.

| **Zutaten** | **INCI-Deklaration** | **Gew.-%** |
|---|---|---|
| Schlick, nativ | Sea Silt | 46,00 |
| Wasser, dem | Aqua demin. | 38,75 |
| Carboxymethylcellulose | Cellulose Gum | 1,10 |
| Glycerol | Glycerin 85% PH.EUR. | 12,00 |
| Iso-Propanol | Isopropyl Alcohol | 2,00 |
| 4-Chlor-m-Kresol | m-Cresol | 0,15 |

Bei der Verwendung von Meeresschlick wird sogenannter nativer sandiger Schlick bevorzugt, der im wesentlichen die folgende Zusammensetzung mineralischer Bestandteile aufweist:
24 bis 56 Gew.-% Sand
70 bis 90 Gew.-% Silt
10 bis 22 Gew.-% Ton.

Neben den mineralischen Anteilen besitzt der sandige Meeresschlick eine Vielzahl von makroskopischen Lebewesen.

Es ist bekannt, daß Meeresschlick Schwefel in Verbindung mit einem ungewöhnlich hohen Anteil an gesättigten und ungesättigten Kohlenwasserstoffen sowie mit geringen Mengen an gesättigten und ungesättigten Fettsäuren aufweist. Die bisher identifizierten Bestandteile des Meeresschlick machen seine sehr gute medizinische Verträglichkeit deutlich.

Die Zusatzstoffe werden in dem vorgenannten Beispiel dem getrockneten und gemahlenen Schlickpulver gemeinsam mit dem Wasser zugesetzt.

Dabei wird dem Wasser, dem. Carboxymethylcellulose als Verdickungsmittel zur Erzielung der gewünschten Konsistenz, Isopropyl Alcohol als Lösungsvermittler und Chlor-Kresol als Antiseptikum zugesetzt.

Zur Herstellung eines geeigneten Verbandsmaterial kann die Schlick-Suspension auf ein größeres flächiges Verbandsgewebe aufgebracht und anschließend zu dem gewünschten Zuschnitt zugeschnitten werden.

Bevorzugt wird die Schlick-Suspension mit 250 bis 750 g/m² Gewebe auf Binden gebracht. Die Binden bestehen bevorzugt aus 70% Cellulose und zu 30% aus Helanka. Das mit der Schlick-Suspension versehene Verbandsmaterial wird in wasserabstoßendes Papier verpackt. Das Papier wird nachfolgend noch einmal in einen Papierbeutel eingeschweißt.

## Patentansprüche

1. Verfahren zur Herstellung einer Schlick-Suspension für ein flächiges Verbandsgewebe, das die folgenden Verfahrensschritte aufweist:
- der Schlick wird auf einer flachen Unterlagen verteilt und an der Luft getrocknet,
- der getrocknete Schlick wird in Stücke gebrochen,
- die Schlickstücke werden mit einer Mahleinrichtung zu Schlickpulver gemahlen,
- das Schlickpulver wird unter Zugabe von Wasser, bevorzugt von gereinigtem Seewasser, in einem vorbestimmten Verhältnis zu einer Suspension verrührt und
- die Suspension wird auf das flächige Verbandsgewebe aufgebracht.

2. Verfahren nach Anspruch 1, **dadurch gekennzeichnet, daß** das flächige Verbandsgewebe als Kompressionsverband bestehend aus Cellulose und einem elastischen Kunststoffanteil ausgebildet ist.

3. Verfahren nach Anspruch 1 oder 2, **dadurch gekennzeichnet, daß** der Schlick in einer Dicke von 0,5 cm bis 4 cm auf der Unterlage verteilt wird, vorzugsweise ungefähr mit einer Dicke von 2 cm.

4. Verfahren nach einem der Ansprüche 1 bis 3, **dadurch gekennzeichnet, daß** der verteilte Schlick für einen halbe bis vier Tage, vorzugsweise für 2 bis 3 Tage bei Umgebungstemperatur getrocknet wird.

5. Verfahren nach einem der Ansprüche 1 bis 4, **dadurch gekennzeichnet, daß** dem Schlick vor dem Verteilen zum Trocknen weitere Substanzen, insbesondere Glyzerin zugesetzt wird.

6. Verfahren nach einem der Ansprüche 1 bis 5, **dadurch gekennzeichnet, daß** dem Schlick nach dem Mahlen weitere Substanzen, insbesondere Glyzerin gemeinsam mit dem Wasser zugesetzt werden.

7. Verfahren nach einem der Ansprüche 1 bis 6, **dadurch gekennzeichnet, daß** die Suspension ein Wasseranteil von 30 bis 50 Gew.-% besitzt.

8. Verbandsgewebe mit einer Schlick-Suspension hergestellt nach einem der Ansprüche 1 bis 7, **dadurch gekennzeichnet, daß** das Verbandsgewebe die Form einer Binde, insbesondere einer Binde für einen Kompressionsverband aufweist.

## Claims

1. A method for preparing a mud suspension for a planar dressing woven fabric, comprising the steps of:
- spreading the mud on a flat substrate and drying it in the air,
- breaking the dried mud into pieces,
- grinding the mud pieces to form a powdered mud, using a grinding device,
- stirring while adding water, preferably purified sea water, to the powdered mud at a predetermined ratio to form a suspension, and
- applying the suspension to the planar dressing woven fabric.

2. The method according to claim 1, **characterized in that** the planar dressing woven fabric is formed as a compression dressing composed of cellulose and an elastic plastic fraction.

3. The method according to claim 1 or 2, **characterized in that** the mud is spread on the substrate at a thickness of 0.5 cm to 4 cm, preferably at a thickness of about 2 cm.

4. The method according to any one of claims 1 to 3, **characterized in that** the mud is dried for half a day to four days, preferably for 2 to 3 days, at an ambient temperature.

5. The method according to any one of claims 1 to 4, **characterized in that** more substances, particularly glycerin, are added to the mud prior to spreading it to make it dry.

6. The method according to any one of claims 1 to 5, **characterized in that** more substances, particularly glycerin, along with the water, are added to the mud after grinding it.

7. The method according to any one of claims 1 to 6, **characterized in that** the suspension has a water fraction of from 30 to 50 % by weight.

8. A dressing woven fabric with a mud suspension as prepared according to any one of claims 1 to 7, **characterized in that** the dressing woven fabric has the shape of a bandage, particularly a bandage for a compression dressing.

## Revendications

1. Procédé de production d'une suspension de pâte pour un bandage mince qui présente les étapes opératoires suivantes :
- la pâte est répartie sur un support plat et séchée à l'air,
- la pâte séchée est cassée en morceaux,
- les morceaux de pâte sont broyés avec un dispositif de broyage en de la poudre de pâte,
- la poudre de pâte est mélangée en ajoutant de l'eau, de préférence de l'eau de mer purifiée, en une suspension dans un rapport prédéterminé et
- la suspension est appliquée sur le bandage mince.

2. Procédé selon la revendication 1, **caractérisé en ce que** le bandage mince est réalisé en tant que bandage de compression composé de cellulose et d'une proportion de plastique élastique.

3. Procédé selon la revendication 1 ou 2, **caractérisé en ce que** la pâte est répartie en une épaisseur allant de 0,5 cm à 4 cm sur le support, de préférence approximativement avec une épaisseur de 2 cm.

4. Procédé selon l'une quelconque des revendications 1 à 3, **caractérisé en ce que** la pâte répartie est séchée pendant une demi-journée à quatre jours, de préférence pendant 2 à 3 jours à température ambiante.

5. Procédé selon l'une quelconque des revendications 1 à 4, **caractérisé en ce que** d'autres substances, notamment de la glycérine, sont ajoutées à la pâte avant la répartition pour le séchage.

6. Procédé selon l'une quelconque des revendications 1 à 5, **caractérisé en ce que** d'autres substances, notamment de la glycérine, sont ajoutées avec l'eau à la pâte après le broyage.

7. Procédé selon l'une quelconque des revendications 1 à 6, **caractérisé en ce que** la suspension possède une proportion d'eau allant de 30 à 50 % en poids.

8. Bandage avec une suspension de pâte produite selon l'une quelconque des revendications 1 à 7, **caractérisé en ce que** le bandage présente la forme d'une bande, notamment d'une bande pour un bandage de compression.
